(19) Europäisches Patentamt

European Patent Office

Office européen des brevets

(11) **EP 4 550 353 A1**

(12) **EUROPEAN PATENT APPLICATION**

(43) Date of publication:
**07.05.2025 Bulletin 2025/19**

(21) Application number: **23207268.6**

(22) Date of filing: **01.11.2023**

(51) International Patent Classification (IPC):
**G16H 50/30** (2018.01)      G16H 50/20 (2018.01)
**G16H 50/70** (2018.01)      A61B 5/00 (2006.01)

(52) Cooperative Patent Classification (CPC):
**G16H 50/30;** A61B 5/7267; G16H 50/20;
G16H 50/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventor: **FENG, Ting
Eindhoven (NL)**

(74) Representative: **Philips Intellectual Property &
Standards
High Tech Campus 52
5656 AG Eindhoven (NL)**

(54) **RISK PREDICTION ABSTENTION IN PATIENT MONITORING**

(57)    A method for real time determination as to whether a prediction model output should be included or withheld from a real time data series based on a combination of a confidence interval associated with the prediction model output and a classification of the prediction model output.

FIG. 3

EP 4 550 353 A1

**Description**

FIELD OF THE INVENTION

The present invention relates to the field of patient monitoring, and in particular to the field of patient monitoring employing machine learning risk prediction models.

BACKGROUND OF THE INVENTION

In practical applications of machine learning models, a decision threshold, which is predetermined through model training data, is utilized to make real-time decisions. Such a model may be further designed to update predictions in response to incoming data. The revised prediction score is then measured against the predetermined threshold to make decisions for current time instances. Consequently, the ultimate product of the machine learning model consists of a series of prediction scores over sequential discrete time instances.

However, the predetermined threshold against which scores are compared is a derivative of model training, and thus is calibrated for optimal performance at a population level. As a result, at an individual level, there can be varying reliability among individual predictions.

For example, a scenario might be considered wherein a clinical decision support algorithm is utilized to evaluate the risk of patients contracting a specific disease. In this scenario, two patients might present identical risk scores, but wherein the confidence levels of the predictions may not be uniform. Consequently, identical risk scores do not necessarily warrant similar treatment for the two patients. Furthermore, if the patient's risk is continuously monitored throughout their hospital stay, the model will revise the risk score in response to new data. In this context, the quality of data instances may vary, leading to fluctuations in prediction confidence at different time instances.

A potential solution to this issue involves estimating prediction confidence in conjunction with the prediction score. However, prediction confidence can be influenced by a variety of factors, and there are various strategies to apply prediction confidence to enhance model performance.

Means for utilizing prediction confidence to improve reliability of data series populated by prediction model outputs would be of value.

SUMMARY OF THE INVENTION

The invention is defined by the claims.

According to examples in accordance with an aspect of the invention, there is provided a computer-implemented method, comprising: receiving input patient data; extracting one or more pre-defined properties of the input data; accessing a machine learning risk prediction model adapted to output a risk score indicative of a risk of a defined adverse clinical event, based on application of the model to an input state model comprising a set of feature vectors; accessing a confidence prediction model (or confidence prediction algorithm or module) adapted to generate a predicted confidence interval for a risk score output by the risk prediction model based on at least the extracted properties of the input data and optionally based on the output risk score of the risk model; updating the feature vectors of the state model based on the received input patient data; executing an instance of the risk prediction model to generate a risk score for the updated state model; executing an instance of the confidence prediction model to generate a confidence interval for the generated risk score; generating a risk classification for the risk score by comparing the risk score against a risk threshold to determine whether the risk threshold is exceeded; determining, based on: the confidence interval and the risk classification, whether to add the generated risk score to a real-time recorded data series, or whether to withhold the generated risk score from the data series.

Embodiments of the invention are based on the concept of using a combination of a confidence interval and a classification of the risk score to determine whether to include or exclude a risk model output as a data point in a data series. By additionally utilizing the risk classification, this allows for example for biasing inclusion/exclusion rules so as to favor either false positives or false negatives in risk assessment. This results in a final data series which is more tuned to a particular clinical context.

In some embodiments, the method further comprises classifying the confidence interval as indicative of high confidence or low confidence in the risk score using a pre-determined one or more criteria. In some embodiments, the determining whether to add or withhold the generated risk score to or from the data series is based in part on the confidence classification.

In some embodiments, the confidence interval may be classified as indicative of low confidence if the confidence interval for the score overlaps with the risk threshold (e.g. if the upper bound of the confidence interval exceeds the risk threshold and/or the lower bound of the confidence interval is smaller than the risk threshold) and high confidence otherwise.

In some embodiments, the determining whether to withhold the generated risk score from the data series is based on deciding to include the risk score in the data series if the risk score does not exceed the risk threshold, and deciding to include the risk score in the data series if the risk score does exceed the risk threshold if and only if the confidence classification is high confidence. This option biases in favor of avoiding false positives in the risk classifications of risk scores in the final data series. As discussed above, the risk classification involves comparing a risk score against a threshold to therefore classify the risk score. By including all risk scores which are below the risk threshold regardless of confidence interval, but excluding those which are above the risk threshold if confidence is low, the final data series will tend to exclude more erroneous data points above the risk threshold than below. This results in a final dataset which is biased in favor of avoiding false risk alerts when evaluating the risk data series.

In some embodiments, the determining whether to withhold the generated risk score from the data series is based on: deciding to include the risk score in the data series if the risk score does exceed the risk threshold, and deciding to include the risk score in the data series if the risk score does not exceed the risk threshold if and only if the confidence classification is high confidence. This option biases in favor of avoiding false negatives in the risk classifications of risk scores in the final data series. In particular, by including all risk scores which are above the risk threshold regardless of confidence interval, but excluding those which are below the risk threshold if confidence is low, the final data series will tend to exclude more erroneous data points below the risk threshold than above. This results in a final dataset which is biased in favor of avoiding missing risk alerts when evaluating the risk data series.

In some embodiments, if the decision is to withhold the prediction score from the data series, the method may further comprises adding a value of the lower bound of the confidence interval for the risk score to the data series in place of the value of the risk score. This results in a final data series in which there are no missing data points, and is biased in favor of avoiding false positives in risk alerts.

In some embodiments, the input patient data includes physiological parameter data, for example received from a set of one or more physiological parameter sensors.

In some embodiments, the extracted properties of the received data include one or more of: a data completeness index, computed based on a percentage of time points in the received data for which data samples are missing; and a data quality index, computed based on applying a pre-defined noise quantification algorithm. In some embodiments, the noise quantification algorithm is adapted to determine a signal to noise ratio of the received data.

In some embodiments, the received patient data includes at least one data series of measurements for a defined clinical parameter, and wherein the received patient data further comprises data context metadata indicative of one or more of: demographic characteristics of the patient and clinical history of the patient.

In some embodiments, the risk prediction model is associated with a target data context profile for input data to which it has been trained to be applied.

In some embodiments, the method further comprises comparing the data context metadata with the target data context profile of the risk prediction model.

In some embodiments, the prediction confidence model is adapted to generate the prediction confidence interval further based on the data context comparison.

In some embodiments, the risk prediction model comprises an ensemble of machine learning algorithms, and wherein each of the machine learning algorithms is trained on a distinct bootstrap-resampled version of an original model training dataset.

In some embodiments, executing an instance of the risk prediction model comprises: applying each of the ensemble of machine learning algorithms to the updated state model; aggregating the outputs of the ensemble of algorithms to derive the output risk score, and computing a spread or variance of the respective outputs of the ensemble of machine learning algorithms to derive a model spread or variance index. In some embodiments, the prediction confidence model may be further adapted to use the spread or variance index as an input.

Another aspect of the invention is a computer program product comprising code means configured, when run on a processor, to cause the processor to perform a method in accordance with any embodiment described in this document or in accordance with any claim of this application.

Another aspect of the invention is a processing device comprising one or more processors adapted to: receive input patient data; extract one or more pre-defined properties of the input data; access a machine learning risk prediction model adapted to output a risk score indicative of a risk of a defined adverse clinical event, based on application of the model to an input state model comprising a set of feature vectors; access a confidence prediction model (or confidence prediction algorithm or module) adapted to generate a predicted confidence interval for a risk score output by the risk prediction model based on at least the extracted properties of the input data and optionally based on the output risk score of the risk model; update the feature vectors of the state model based on the received input patient data; execute an instance of the risk prediction model to generate a risk score for the updated state model; execute an instance of the confidence prediction model to generate a confidence interval for the generated risk score; generate a risk classification for the risk score by comparing the risk score against a risk threshold to determine whether the risk threshold is exceeded; and determine, based on: the confidence interval and the risk classification, whether to add the generated risk score to a real-time recorded data series, or whether to withhold the generated risk score from the data series.

In some embodiments, the one or more processors may be further adapted to: classify the confidence interval as indicative of high confidence or low confidence in the risk score using a predetermined one or more criteria, and wherein the determining whether to add or withhold the generated risk score to or from the data series is based in part on the confidence classification.

In some embodiments, the determining is based on: deciding to include the risk score in the data series if the risk score does not exceed the risk threshold, and deciding to include the risk score in the data series if the risk score does exceed the risk threshold if and only if the confidence classification is high confidence.

In some embodiments, the determining is based on: deciding to include the risk score in the data series if the risk score does exceed the risk threshold, and deciding to include the risk score in the data series if the risk score does not exceed the risk threshold if and only if the confidence classification is high confidence.

Another aspect of the invention is a system comprising: a processing device in accordance with any embodiments described in this document or in accordance with any claim; and a set of physiological parameter sensors operatively coupled to the processing device for supplying at least a subset of the input patient data.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 outlines steps of an example method in accordance with one or more embodiments of the invention;
Fig. 2 is a block diagram of an example processing device and system in accordance with one or more embodiments of the invention;
Fig. 3 is a block diagram illustrating a processing flow in accordance with one or more embodiments;
Figs. 4-6 shows a set of graphs illustrating different example rules or strategies for determining inclusion or exclusion of risk score points in a data series.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provide a method for real time determination as to whether a prediction model output should be included or withheld from a real time data series based on a combination of a confidence interval associated with the prediction model output and a classification of the prediction model output.

For example, according to at least one advantageous set of embodiments, there may be a provided a system that enables real-time prediction abstention based on confidence measurements. The system may be adapted to operate in real-time such that, whenever new data comes in, a chain of computations and processes will be triggered. Upon receipt of new data, the system may update its state model, where the state model stored feature vectors for calculating risk scores. In the meantime, a process will evaluate properties of the incoming data, which will serve as one of the source information to calculate prediction confidence. The state model with feature vectors may feed into two parallel processes: the first process calculates a risk score using a pre-trained machine learning model saved in the system, and the second process estimates a prediction confidence, for example based on both model behavior and incoming data properties. Finally, a process may gather confidence information and make a decision as to whether the predicted risk score should be withheld or not.

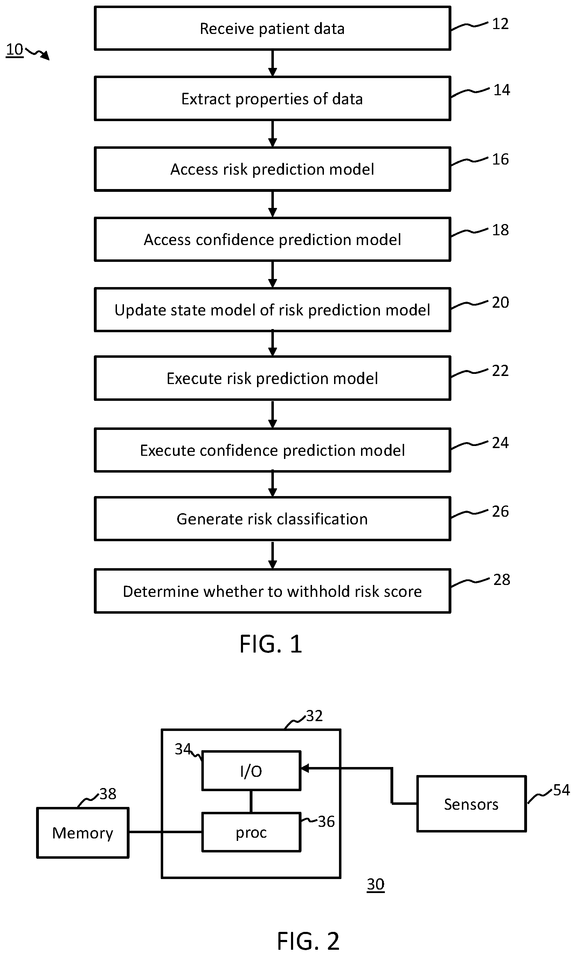

Fig. 1 outlines in block diagram form steps of an example method according to one or more embodiments. The steps will be recited in summary, before being explained further in the form of example embodiments.

The method 10 comprises receiving 12 input patient data. By way of example, the input patient data may include physiological parameter data, for example received from a set of one or more physiological parameter sensors. The input patient data may comprise one or more real time data streams. The input patient data may include at least one data series of measurements for a defined clinical parameter. The input patient data may further comprise metadata. The metadata may include data context metadata. In some embodiments, the context metadata may include demographic characteristics of the patient and/or clinical history of the patient.

The method may further comprise extracting 14 one or more pre-defined properties of the input data. By way of example, in some embodiments, the extracted properties of the received data include one or more of: a data completeness index, for example computed based on a percentage of time points in the received data for which data samples are missing, and/or a data quality index, for example computed based on applying a pre-defined noise quantification algorithm.

The method further comprises accessing 16 a machine learning risk prediction model adapted to output a risk score indicative of a predicted risk of a defined adverse clinical event. This may be based on application of the model to an input state model comprising a set of feature vectors.

The method further comprises accessing 18 a confidence prediction model (or confidence prediction algorithm or module) adapted to generate a predicted confidence interval for a risk score output by the risk prediction model. The generation of the confidence interval may be based on at least the extracted properties of the input data and/or based on the output risk score of the risk model and/or based on the updated state model.

The method may further comprise updating 20 the feature vectors of the state model based on the received input patient data. A state model in machine learning represents the system at any given point in time using a set of variables, or "features". In the context of patient data, these features could be various health indicators such as any of heart rate, blood pressure, temperature, lab results, etc.

The method may further comprise executing 22 an instance of the risk prediction model to generate a risk score for the updated state model.

The method may further comprise executing 24 an instance of the confidence prediction model to generate a confidence interval for the generated risk score. Executing an instance of the confidence prediction model may comprise executing one or more algorithms comprised by the confidence prediction model.

The method may further comprise generating 26 a risk classification for the risk score by comparing the risk score against a risk threshold to determine whether the risk threshold is exceeded.

The method may further comprise determining 28, based on: the confidence interval and the risk classification, whether to add the generated risk score to a real-time recorded data series, or whether to withhold the generated risk score from the data series.

If the decision is to add the generated risk score to a real-time recorded data series, the method may further comprise a set of adding the generated risk score to the real-time recorded data series. This data series may be stored in a local memory or may be stored at a location remote from the processor executing the method.

As noted above, the method 10 can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the system 30 further comprises a set of one or more physiological parameter sensors 54 operatively coupled to the processing device 32 for supplying at least a subset of the input patient data.

The system 30 may further comprise a memory 38 for storing computer program code (i.e. computer-executable code) which is configured for causing the one or more processors 36 of the processing device 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

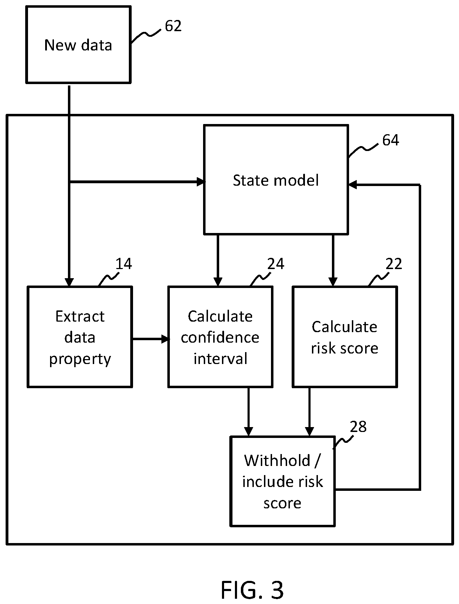

Fig. 3 schematically outlines the data processing flow in accordance with one or more embodiments. The method comprises receiving new data 62. The new data 62 is used to update the state model 64 of the risk prediction model, and, in parallel, a set of pre-defined data properties are extracted 14 from the data. The method further comprises applying the risk model to calculate a risk score 22 in accordance with the updated state model 64. The method further comprises computing 24 a confidence interval for the generated risk based on the extracted properties 14 of the input data and optionally based on the risk score and/or the state model. As illustrated in Fig. 3, the calculation of the confidence interval may be based on the updated state model 64, e.g. in a case where the confidence interval is computed based on applying an ensemble of risk prediction models to the same state model and computing statistical information about the outputs (see description later).

The method further comprises deciding 28 whether to withhold (abstain) the risk prediction score 22, based on the confidence interval and the risk score 22. For example, the risk score may be classified based on whether it exceeds one or more thresholds and the abstention decision 28 may be made based on the confidence interval and the risk classification.

A part of the method involves estimating prediction confidence 24. This feature will now be discussed in more detail.

For estimating prediction confidence, there are in general multiple sources of potential unreliability of risk scores in a real-time machine learning prediction system.

For example, data is received in in a continuous fashion. However, some of the data that are required to calculate features as input to the machine learning model may be missing. This data capture problem is common in e.g. a real-world application where incoming data is from wearable devices where the data completeness can be easily affected by user behavior. Such a data capture problem is also common in e.g. in a clinical decision support system where certain physiological measurements are only accessed when deemed to be medically necessary.

To account for this, in some embodiments, the extracted properties of the received data may include: a data completeness index, computed based on a percentage of time points in the received data for which data samples are missing.

For example, data availability might be quantified as percentage of time instances that the data is missing, factoring in the sampling frequency of sensing devices (or other data generation devices).

By way of example, a data completeness index may be assigned a value between 0 and 1, where , with 1 being 100% complete and 0 being no data available.

A further source of unreliability may be noise in the incoming data. For example, wearable devices may sometimes report measurements outside of physiological range. By way of further example, measurements even from gold-standard clinical devices may be affected by patient motion (motion artefacts).

Thus, to account for this, in some embodiments, the extracted properties of the received data may include a data quality index, computed based on applying a pre-defined noise quantification algorithm.

By way of example, there are several standard types of noise quantification algorithms that could be applied to input data. One example is a signal to Noise Ratio (SNR) quantification algorithm. Such an algorithm works by measuring the proportion of signal power to noise power within a given dataset. A higher SNR indicates less noise compared to the signal. In the context for example of wearable devices, this algorithm can be used to assess the reliability of measurements by comparing the strength of the physiological signals (like heart rate or body temperature) to the noise that may be caused by phenomena such as motion artifacts. Another example is a standard deviation and/or variance computation algorithm. These are statistical measures of dispersion or spread, which can also be used to quantify noise. If the data is normally distributed, then a higher standard deviation or variance indicates higher noise. In the wearable context, these measures could be used to assess the reliability of measurements over time - for example, if the heart rate measurements vary widely from one moment to the next, this could indicate the presence of noise.

Another possibility is to apply spectral analysis. This involves analyzing the frequency components of a signal. Noise often manifests as high-frequency components, so spectral analysis may be applied to identify and quantify noise in the data by determining the relative strength of frequency components in the signal which are known to be associated with error or noise. In the context of wearable devices, this could be used to filter out high-frequency noise from the measured signals.

Various example noise quantification algorithms are known, for example auto-correlation function, or even machine learning techniques.

Data quality might also be estimated as the percentage of measurements that are outside of a plausible physiological range. To implement this, a plausible physiological range may be pre-defined, for example based on statistical analysis of population data, and measured values of a physiological parameter compared against the plausible range.

Whichever method is used, the data quality index may be assigned a value from 0 to 1, with 1 corresponding to highest quality and 0 being lowest.

Finally, incoming data may be associated with a different clinical context compared with the data used to train the risk prediction machine learning model. For example, in the case of a clinical decision support application, the incoming data can originate from patients in different acuity units. In the case of data received from wearable devices, the subject may be under different physical activity states (e.g. exercising versus sleeping).

In some embodiments, this may be accounted for in the computed confidence score or confidence interval by comparing the context associated with the received data with the context associated with the risk prediction model.

For example, to state this more concretely, in some embodiments, the received patient data may include at least one data series of measurements for a defined clinical parameter, and wherein the received patient data further comprises data context metadata indicative for example of one or more of: demographic characteristics of the patient and clinical history of the patient. By way of further example, other possible relevant data context properties may include: patient current medications, patient lifestyle factors (e.g. smoking, alcohol use, or physical activity levels), time of day of data collection, patient's physical condition at the time of data collection (e.g. at rest or exercising), environmental conditions (e.g. temperature or humidity).

The risk prediction model may be associated with a target data context profile for input data to which it has been trained to be applied. A machine learning model is trained using a specific set of data having a particular "context profile", by which is meant that the data was associated with certain contextual factors. This could include factors such as the demographic characteristics of the patients, their clinical history, and the specific conditions under which the data was collected. A model is designed to make predictions based on input data that fits this context profile.

The method may further comprise comparing the data context metadata with the target data context profile of the risk prediction model. To make comparison more reliable, the two sets of contextual information might in some examples be transformed into numerical values or vectors. For example, demographic characteristics such age and sex could be represented as numerical values, while more complex data such as clinical history could be encoded as a vector of binary or categorical values (e.g., 1 if a certain medical condition is present, 0 if not). Once the context data has been so encoded, it can then be compared using various statistical measures or distance metrics, depending on the nature of the data.

The prediction confidence model may be further adapted to generate the prediction confidence interval further based on the data context comparison.

For example, a data context similarity index might be determined, with a value between 0 and 1, where 0 represents zero similarity and 1 represents maximum similarity.

Another source of possible unreliability of model prediction outputs lies within the trained model itself. Machine learning models are typically trained using data obtained from a patient cohort, where some degree of heterogeneity among individuals is unavoidable. Therefore a small perturbation of the training cohort may result in slightly different prediction scores for an individual case. This creates an uncertainty of model output, which can be formulated into confidence measurements.

By way of example, this source of potential model uncertainty can be estimated by training an ensemble of models with different cohort subsets.

By way of example, in some embodiments, the risk prediction model may comprise an ensemble of machine learning algorithms, and wherein each of the machine learning algorithm is trained on a distinct bootstrap-resampled version of an original model training dataset. Executing an instance of the risk prediction model may comprise: applying each of the ensemble of machine learning algorithms to the updated state model; aggregating the outputs of the ensemble of algorithms to derive the output risk score; and computing a spread or variance of the respective outputs of the ensemble of machine learning algorithms to derive a model spread or variance index. The prediction confidence model may be further adapted to use the spread or variance index as an input.

Thus, in summary, data availability, data quality, data context, and model behavior are all factors that impact prediction confidence. To capture these elements and convert them into confidence measurements, the various techniques outlined above can be used.

For computing/predicting a confidence interval from one or more of the various factors mentioned above, different methods are possible.

One approach is to first compute an index, for example between 0 and 1, for the respective indicator, e.g. data completeness, data quality, or data context, where 0 represents zero confidence and 1 represents 100% confidence, and then to map this index to a confidence interval based on a pre-determined transfer function.

Indices derived from a plurality of different factors might be combined in some examples, e.g. an average or weighted average taken, before a confidence interval is then computed based on the combination.

Assuming that a single confidence index is arrived at through one or more of the above techniques, and optionally with multiple indices aggregated into a single index, a process for converting from this confidence index to a confidence interval will now be discussed.

The risk score output by the risk prediction model, and for which the confidence interval is to be derived, may be denoted by RS.

In addition, a confidence interval multiplier (CIM) may be defined which is a multiplier for use in tuning the range of the confidence interval based on the confidence index, as discussed below.

In general terms, a confidence interval represents a range of values (about a central point defined by the output risk score) within which it is expected that a notional 'true value' of the risk score lies, to a certain level of confidence. It will be recognized that, as a general principle, the confidence interval should be wider where there is lower confidence in the risk score, and narrower where there is higher confidence in the risk score. The amount of confidence in the score may be quantified by the already computed confidence index, having a value between 0 and 1, with 0 representing a minimum confidence and 1 representing a high confidence. It is necessary to define a function that maps between the index and a range for the confidence interval.

The range of values spanned by the confidence interval may be defined as a certain percentage of the value of the risk score, RS. One way to define the transfer function is therefore to set a percentage interval to which a minimum confidence index (0) should correspond and a percentage interval to which a maximum confidence interval (1) should correspond.

For instance, to take an example, an overall Confidence Index (CI) might be computed from one or more of the factors previously mentioned, e.g. from Data Completeness and Data Quality indices, having a value between 0 and 1.

It is possible to define a function which translates this confidence index, CI, into a confidence interval multiplier (CIM).

For example, a CI of 1 (highest confidence) might be defined as corresponding to a 5% interval around the Risk Score and a CI of 0 (lowest confidence) might be defined as corresponding to a 50% interval (wherein the percentage intervals can be defined as desired). In this case, a linear function between CI and CIM might be as follows:

$$CIM = [0.45 \times (1 - CI)] + 0.05$$

In this case, if CI is 1, CIM becomes 0.05. If CI is 0, CIM becomes 0.50.

The Confidence Interval (bounds) may then be defined as:

$$Lower\ Bound = RS - (RS \times CIM)$$

$$Upper\ Bound = RS + (RS \times CIM)$$

To generalize the formula for any arbitrary percentage interval at maximum and minimum confidence indices, the following procedure may be followed.

Let MaxInt be the desired percentage interval (expressed as a fraction, e.g., 5% = 0.05) for the maximum confidence index (CI = 1) and MinInt be the desired percentage interval for the minimum confidence index (CI = 0).

A linear relationship between the confidence interval multiplier (CIM) and the Confidence Index (CI) may be represented by:

$$CIM = [(MinInt - MaxInt) \times (1 - CI)] + MaxInt$$

Using this value, the boundaries of the confidence interval may be defined by:

$$Lower\ Bound = RS - (RS \times CIM)$$

$$Upper\ Bound = RS + (RS \times CIM)$$

The values for MaxInt and MinInt can be tuned as desired.

Thus, the confidence prediction model may comprise one or more algorithms which are configured to compute one or more confidence indices based on the different factors mentioned previously, and then to compute the confidence interval for example using the techniques discussed above.

As mentioned above, if more than one method or factor is used to determine the confidence interval, the final confidence range can be derived by combining the results. There are multiple ways to do this. One approach is to use weighted averaging, wherein different weights are assigned to the CI indices derived from different methods, for example based on their perceived importance. Another approach is to only consider the intersection of CI indices obtained from the different methods. Another approach is to consider the union of CI indices from all methods, leading to a broader CI.

It will be noted that one of the above-described methods for evaluating confidence was based on statistical information derived from outputs of an ensemble of risk prediction models. In some embodiments, the output of this approach may be used by the confidence prediction model to directly compute a confidence interval. For example, the method may comprise executing the risk prediction model using an ensemble of different ML algorithms or variations of the same algorithm. The set of predicted risk scores is collected. The mean of the risk scores is computed. The spread or variance of the predicted risk scores is also calculated from the ensemble. This may for example be the standard deviation of the predictions. A confidence interval may then be computed by using the standard deviation (or another metric of spread) to compute the confidence interval. For example, for a 95% confidence interval in a normal distribution, the interval may be computed as mean$\pm 1.96 \times$standard deviation.

As discussed above, embodiments of the invention involve determining, based on the confidence interval and the risk classification, whether to add the generated risk score to a real-time recorded data series, or whether to withhold the generated risk score from the data series.

By way of background, for real-time machine learning applications, it is important to allow abstention of unreliable predictions to avoid making prediction mistakes. However, there is a natural trade-off between prediction performance and prediction abstention. For example, if a system is designed to be conservative so as to withhold predictions that do not meet a very high confidence level, then no decisions can be made for those time instances where the prediction is withheld.

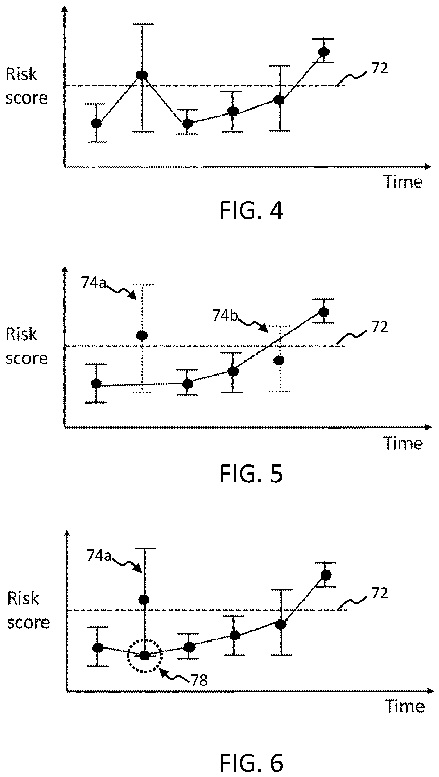

There will now be discussed three possible prediction abstention strategies with reference to Figs. 4-6. Each of Figs. 4-6 schematically illustrate a respective abstention strategy. Each black dot represents a prediction score generated by the risk prediction model. These scores are presented on a time series chart, where the y-axis represents risk score and the x-axis represents time. The bracket around each risk score point represents the confidence interval generated by the confidence prediction model for each risk score. The dashed line 72 represents a risk threshold, wherein classification of risk scores is performed based on whether the score exceeds the threshold. For example, risk scores which exceed which exceed the risk threshold 72 are classified as indicating high risk for the relevant adverse clinical event, and risk scores which do not exceed the risk threshold 72 are classified as low risk or neutral risk for the relevant adverse clinical event.

With regards to the confidence interval, this represents the range of values that is likely to contain a notional 'true value' of the risk score to a certain level of confidence, for example 95%.

The confidence interval for each risk score may in some embodiments be classified as being indicative of high confidence or low confidence in the risk score. For example, the confidence interval may be classified as indicative of low confidence if the confidence interval for the score overlaps with the risk threshold and high confidence otherwise.

In a first abstention strategy, illustrated by Fig. 4, no risk scores are actually withheld from the compiled real-time data series, but instead, the confidence interval of each risk score is presented to the end user via a user interface. This strategy might be favored if it is preferred that clinical users make the final judgement on each prediction instance. The user interface may provide the user an option to remove any risk score from the data series.

A second abstention strategy, illustrated by Fig. 5, is to abstain all risk scores for which the confidence interval is classified as indicative of low confidence, e.g. those for which the confidence interval overlaps with the risk threshold 72. The withheld risk scores are indicated at arrows 74a and 74b. Thus, the abstention is determined solely based on the confidence level, and independent of the value of the risk score itself. This is the most aggressive abstention strategy, and results in the greatest number of risk score abstentions.

The third strategy, as illustrated by Fig. 6, is to withhold (or adjust) low-confidence prediction only if the prediction score is a selected one of: above the predetermined decision threshold or below the predetermined decision threshold. This is the preferred strategy in accordance with embodiments of the present invention.

This partial abstention strategy is targeted to either decrease false positives (when abstaining low-confidence prediction scores that are above the risk decision threshold 72) or decrease false negatives (when abstaining low-confidence prediction scores that are below the risk decision threshold 72). Which bias is used may be chosen as desired, for example based on clinical preferences of the end user, or based on a known underlying bias of the real-world data. In the latter case, for example for a binary classification application, if the true positive events are rare compared with true negative events, then it is most optimal to partially abstain positive cases - i.e. those prediction scores with low-confidence and which are above the predetermined decision threshold.

If the aim is to decrease false positives, the method may thus comprise deciding to include the risk score in the data series in all cases where the risk score does not exceed the risk threshold 72, and deciding to include the risk score in the data series in cases where the risk score does exceed the risk threshold if and only if the confidence classification is high confidence. This approach is illustrated in Fig. 6 which shows that risk score 74a has been withheld since the risk score is above the risk threshold 72 but the confidence interval overlaps with the risk threshold 72 and thus is classified as indicating low confidence.

Conversely, if the aim is to decrease false negatives, the method may comprise deciding to include the risk score in the data series in all cases where the risk score does exceed the risk threshold, and deciding to include the risk score in the data series in cases where the risk score does not exceed the risk threshold if and only if the confidence classification is high confidence. If this strategy were followed, then risk score 74a would be included in the data series, but risk score 74b would not.

In some embodiments, this strategy might be combined with an information preservation strategy, wherein partial information about the abstained risk scores is preserved in the data series, such that decisions can still be made. For example, in some embodiments, the lower bound of the confidence interval might be used to guide decisions instead of the low-confidence prediction score. In other words, if the decision is to withhold the prediction score from the data series, the method may further comprise adding a value of the lower bound of the confidence interval for the risk score to the data series in place of the value of the risk score. This is illustrated in Fig. 6 which shows the lower bound 78 of the confidence interval of the withheld risk score 74a added to the data series.

In some embodiments, the method may further comprise configuring or tuning the confidence level represented by the confidence interval. In other words, if the confidence interval represents a range of values within which there is x% confidence that the true risk score lies, then the value of x may be adjusted in accordance with some embodiments. The particular abstention strategy used may be selected by the user in some embodiments. The user may select between the partial abstention strategy favoring false positives or favoring false negatives in some embodiments.

The confidence interval width (in percentage terms) may be tuned as desired to weight in favor of more precise or less precise prediction. This may be customized based on user requirements and/or based on optimizing one or more user performance metrics. It can also be tuned in a supervised fashion, e.g. in a case where a specific targeted performance metric is available, or if the abstention strategy/threshold needs to be different under different contextual settings. For example, increasing the confidence interval width may result in increased AUROC (Area Under the Receiver Operating Characteristic curve) and Average Precision. These are two commonly used metrics in binary classification tasks.

As mentioned above, one part of the method may be updating the state model of the risk prediction model.

A state model in machine learning represents the system at any given point in time using a set of variables, or "features". In the context of patient data, these features could be various health indicators such as heart rate, blood pressure, temperature, lab results, etc.

When new patient data is received, the feature vectors of the state model may be updated according to the following general steps. A first step may be data preprocessing. This step involves preparing the new data so it can be used in the model. This may include for example cleaning the data (e.g., handling missing values), normalizing or standardizing numerical data, and/or encoding categorical data. A second step may be vector representation. In this step, the preprocessed data may be converted into a format that can be understood by the model. In machine learning, this may comprise converting the data into a vector, or an array of numbers. Each element of the vector may correspond to a feature. A third step may be to implement model update. Here, once the new data has been converted into a feature vector, it can be used to update the state model. The exact process for this will depend upon the type of model. For example, if the model is a type of neural network, the weights of the network might be updated through backpropagation using the new data. If the model is a type of Bayesian model, the new data might be used to update the prior probabilities. A final possible step may be re-evaluation. Here, after the model has been updated, its performance may optionally be re-evaluated to ensure that the update has improved the model. This may involve using a validation dataset which is previously unseen by the model and comparing the model's predictions to the ground truth.

As discussed above, embodiments of the invention employ a machine learning risk prediction model adapted to output a risk score indicative of a risk of a defined adverse clinical event, based on application of the model to an input state model comprising a set of feature vectors. The method involves updating the feature vectors of the state model based on received input patient data (e.g. real time patient physiological parameter data e.g. received from sensors or wearable devices), and executing an instance of the risk prediction model to generate a risk score for the updated state model.

Developing such a risk prediction model may involve for example steps of: data gathering, data preprocessing, model selection, model training and model evaluation.

With regards to data gathering, for a risk prediction model, the training data should preferably include all relevant patient data that will be available during real-world execution of the model, and which could be indicative of the risk of the relevant adverse clinical event. This data could be collected from various sources such as electronic health records, wearable devices, lab test results, etc. The data may be time-series data, thereby permitting training of a real-time prediction model. The data also should include information about the occurrence of the target adverse clinical event, so that the model can learn associations between data patterns and the occurrence of the adverse clinical event.

Once the data has been collected, there may be applied a step of data preprocessing. This might typically involve surveying the data to identify any missing data, normalizing any numerical data to ensure all data entries are comparable, encoding categorical data, and creating new feature vectors. In the vase of time-series data, the data may be segmented into discrete time windows in some examples.

With regards to the model, a variety of machine learning model types may be suitable for predicting risk based on patient data. Some options include: logistic regression mode, random forest model, gradient boosting machines, an artificial neural network, or support vector machines. With regards to neural networks, there are a variety of particular types of neural network that might be considered. These include for example Recurrent Neural Networks (RNN) or Long Short-Term Memory (LSTM) networks. These are both particularly suited to handling time-series data and are able to recognize patterns over time.

Taking by way of example an LSTM mode, the architecture of the LSTM model could include an input layer, one or more LSTM layers, and an output layer.

With regards to training the mode, optionally, the compiled training data may be split into a training set and a validation set. The model may then be trained using the training set, and the validation set used to monitor the model's performance at later points.

As is well known, model training is performed by feeding the model input data (e.g. patient feature vectors) and the target data (occurrence of the clinical event). The model generates predictions based on the input data, and the optimization algorithm acts to adjusts the model parameters to minimize the difference between the predictions and the target data as defined by a loss function.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method, comprising:

   receiving input patient data;
   extracting one or more pre-defined properties of the input data;
   accessing a machine learning risk prediction model adapted to output a risk score indicative of a risk of a defined adverse clinical event, based on application of the model to an input state model comprising a set of feature vectors;
   accessing a confidence prediction model adapted to generate a predicted confidence interval for a risk score output by the risk prediction model based on at least the extracted properties of the input data;
   updating the feature vectors of the state model based on the received input patient data;
   executing an instance of the risk prediction model to generate a risk score for the updated state model;
   executing an instance of the confidence prediction model to generate a confidence interval for the generated risk score;
   generating a risk classification for the risk score by comparing the risk score against a risk threshold to determine whether the risk threshold is exceeded;
   determining, based on: the confidence interval and the risk classification, whether to add the generated risk score to a real-time recorded data series, or whether to withhold the generated risk score from the data series.

2. The method of claim 1, further comprising classifying the confidence interval as indicative of high confidence or low confidence in the risk score using a pre-determined one or more criteria, and wherein the determining whether to add or withhold the generated risk score to or from the data series is based in part on the confidence classification.

3. The method of claim 2, wherein the confidence interval is classified as indicative of low confidence if the confidence interval for the score overlaps with the risk threshold and high confidence otherwise.

4. The method of claim 2 or 3, wherein the determining is based on:

> deciding to include the risk score in the data series if the risk score does not exceed the risk threshold, and deciding to include the risk score in the data series if the risk score does exceed the risk threshold if and only if the confidence classification is high confidence;
> **OR**
> deciding to include the risk score in the data series if the risk score does exceed the risk threshold, and deciding to include the risk score in the data series if the risk score does not exceed the risk threshold if and only if the confidence classification is high confidence.

5. The method of any preceding claim, wherein, if the decision is to withhold the risk score from the data series, the method further comprises adding a value of the lower bound of the confidence interval for the risk score to the data series in place of the value of the risk score.

6. The method of any of claims 1-5, wherein the input patient data includes physiological parameter data, for example received from a set of one or more physiological parameter sensors.

7. The method of any of claims 1-6, wherein the extracted properties of the received data include one or more of:

> a data completeness index, computed based on a percentage of time points in the received data for which data samples are missing;
> a data quality index, computed based on applying a pre-defined noise quantification algorithm.

8. The method of claim 7, wherein the noise quantification algorithm is adapted to determine a signal to noise ratio of the received data.

9. The method of any of claims 1-8,

> wherein the received patient data includes at least one data series of measurements for a defined clinical parameter, and wherein the received patient data further comprises data context metadata indicative of one or more of: demographic characteristics of the patient and clinical history of the patient, and
> wherein the risk prediction model is associated with a target data context profile for input data to which it has been trained to be applied, and
> wherein the method further comprises comparing the data context metadata with the target data context profile of the risk prediction model, and
> wherein the prediction confidence model is adapted to generate the prediction confidence interval further based on the data context comparison.

10. The method of any of claims 1-9,

> wherein the risk prediction model comprises an ensemble of machine learning algorithms, and wherein each of the machine learning algorithms is trained on a distinct bootstrap-resampled version of an original model training dataset, and
> wherein executing an instance of the risk prediction model comprises:

> > applying each of the ensemble of machine learning algorithms to the updated state model,
> > aggregating the outputs of the ensemble of algorithms to derive the output risk score, and
> > computing a spread or variance of the respective outputs of the ensemble of machine learning algorithms to derive a model spread or variance index; and

> wherein the prediction confidence model is further adapted to use the spread or variance index as an input.

11. A computer program product comprising code means configured, when run on a processor, to cause the processor to perform a method in accordance with any of claims 1-10.

12. A processing device comprising one or more processors adapted to:

> receive input patient data;
> extract one or more pre-defined properties of the input data;
> access a machine learning risk prediction model adapted to output a risk score indicative of a risk of a defined adverse clinical event, based on application of the model to an input state model comprising a set of feature vectors;
> access a confidence prediction model adapted to generate a predicted confidence interval for a risk score output by the risk prediction model based on at least the extracted properties of the input data;
> update the feature vectors of the state model based on the received input patient data;
> execute an instance of the risk prediction model to generate a risk score for the updated state model;
> execute an instance of the confidence prediction model to generate a confidence interval for the generated risk score;
> generate a risk classification for the risk score by comparing the risk score against a risk threshold to determine whether the risk threshold is exceeded;
> determine, based on: the confidence interval and the risk classification, whether to add the generated risk score to a real-time recorded data series, or whether to withhold the generated risk score from the data series.

13. The processing device of claim 12, wherein the one or more processors are further adapted to: classify the confidence interval as indicative of high confidence or low confidence in the risk score using a pre-determined one or more criteria, and wherein the determining whether to add or withhold the generated risk score to or from the data series is based in part on the confidence classification.

14. The processing device of claim 13, wherein the determining is based on:

> deciding to include the risk score in the data series if the risk score does not exceed the risk threshold, and deciding to include the risk score in the data series if the risk score does exceed the risk threshold if and only if the confidence classification is high confidence;
> **OR**
> deciding to include the risk score in the data series if the risk score does exceed the risk threshold, and deciding to include the risk score in the data series if the risk score does not exceed the risk threshold if and only if the confidence classification is high confidence.

15. A system comprising:

> a processing device as claimed in claim 14; and
> a set of physiological parameter sensors operatively coupled to the processing device for supplying at least a subset of the input patient data.

FIG. 1

FIG. 2

FIG. 3

FIG. 4

FIG. 5

FIG. 6

| | Europäisches Patentamt European Patent Office Office européen des brevets | **EUROPEAN SEARCH REPORT** | Application Number EP 23 20 7268 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/120017 A1 (DAWNLIGHT TECH INC [US]) 9 June 2022 (2022-06-09) | 1,2,4-9, 11-15 | INV. G16H50/30 |
| Y | * figures 1-7 * <br> * page 1, paragraph 0003 - page 16, paragraph 0075 * <br> * page 18, paragraph 0092 - page 33, paragraph 00144 * <br> ----- | 3,10 | ADD. G16H50/20 G16H50/70 A61B5/00 |
| X | US 2022/044818 A1 (CHANG YALE [US]) 10 February 2022 (2022-02-10) | 1-15 | |
| Y | * the whole document, in partocular paragraphs [0004]-[0045], [0079]-[0083], and figures 1-6 * <br> ----- | 3,10 | |
| Y | LOTTA MEIJERINK ET AL: "Uncertainty estimation for classification and risk prediction on medical tabular data", ARXIV.ORG, CORNELL UNIVERSITY LIBRARY, 201 OLIN LIBRARY CORNELL UNIVERSITY ITHACA, NY 14853, 23 May 2020 (2020-05-23), XP081668644, * the whole document * <br> ----- | 10 | |
| A | Anonymous: "Confidence interval - Wikipedia", , 25 September 2023 (2023-09-25), XP093141750, Retrieved from the Internet: URL:https://en.wikipedia.org/w/index.php?t itle=Confidence_interval&oldid=1176947832 [retrieved on 2024-03-15] * the whole document * <br> ----- | 1-15 | TECHNICAL FIELDS SEARCHED (IPC) <br> G16H A61B |

The present search report has been drawn up for all claims

| Place of search | Date of completion of the search | Examiner |
|---|---|---|
| Munich | 21 March 2024 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 20 7268

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2024

| Patent document cited in search report | Publication date | Patent family member(s) | Publication date |
|---|---|---|---|
| WO 2022120017 A1 | 09-06-2022 | NONE | |
| US 2022044818 A1 | 10-02-2022 | NONE | |

EPO FORM P0459

For more details about this annex : see Official Journal of the European Patent Office, No. 12/82